# EUROPEAN PATENT APPLICATION

(11) **EP 4 478 593 A1**
(43) Date of publication of application: **18.12.2024**
(21) Application number: 22937179.4
(22) Date of filing: 11.10.2022
(51) Int. Cl.: H02M 1/08, A61B 10/02, H02M 7/04

(54) **HIGH-FREQUENCY CIRCUIT AND SYSTEM FOR CRYOGENIC BIOPSY**

(30) Priority: 15.04.2022 CN 202210396885
(71) Applicant: Shanghai Cultiva Medical Device Co., Ltd., Shanghai 200120 (CN)
(72) Inventor: KONG, Fanbin, Shanghai 201210 (CN); SUN, Xiaoan, Shanghai 201210 (CN); HE, Yihui, Shanghai 201210 (CN)
(74) Representative: Argyma
(86) International application number: PCT/CN2022/124681
(87) International publication number: WO 2023/197542

(57) **Abstract**

The present invention belongs to the technical field of medical instruments, and in particular to a high-frequency circuit and system for a low-temperature biopsy, which can be applied to a breast biopsy system. The circuit includes: a control circuit and an amplification circuit, wherein the amplification circuit includes: an amplification unit and a high-frequency power transistor. An input end of the amplification unit is connected to an output end of the control circuit, to receive a high-frequency driving signal generated by the control circuit. An output end of the amplification unit is connected to a base of the high-frequency power transistor. A collector of the high-frequency power transistor is a high-frequency energy output end or connected to a high-frequency energy output end. The high-frequency energy output end outputs high-frequency energy. According to the present invention, the problems of high mechanical cutting process requirements, high costs and easy bleeding in the prior art can be solved, and the problems that a traditional high-frequency electrotome has a large thermal damage depth, is easy to damage taken-out tissue and affects a pathological analysis result can be solved.

## Description

### TECHNICAL FIELD

The present invention relates to the technical field of medical instruments, and in particular to a high-frequency circuit and system for a low-temperature biopsy.

### BACKGROUND

In China, the health burden caused by cancers is increasing year by year. Every year, more than 1.6 million people are diagnosed with cancers, and 1.2 million people die due to cancers. As the incidence of breast cancers continues to rise in China, it is urgent to improve the detection, diagnosis, and treatment of the breast cancers.

At present, mainstream products for a breast biopsy mainly use open sampling provided by Mammotome and closed sampling provided by EnCor, both of which are mechanical cutting methods.

Mechanical cutting has the following disadvantages: on the one hand, since a minimally invasive sampling needle is small, the machining process requirements are high and the costs are also high; and on the other hand, mechanical cutting inevitably leads to bleeding, which brings certain harm and risk to a patient.

A high-frequency electrotome is used in other surgery. Although the high-frequency electrotome has a function of electric cutting and coagulation, a thermal damage depth is large, which may damage taken-out tissue and affect a pathological analysis result when applied to the biopsy.

### SUMMARY

The present invention provides a high-frequency circuit and system for a low-temperature biopsy, to solve the problems of high mechanical cutting process requirements, high costs and easy bleeding in the prior art, and the problems that a traditional high-frequency electrotome has a large thermal damage depth, is easy to damage taken-out tissue and affects a pathological analysis result.

According to a first aspect of the present invention, provided is a high-frequency circuit for a low-temperature biopsy, including a control circuit and an amplification circuit, wherein
the amplification circuit includes: an amplification unit and a high-frequency power transistor;
an input end of the amplification unit is connected to an output end of the control circuit, to receive a high-frequency driving signal generated by the control circuit, an output end of the amplification unit is connected to a base of the high-frequency power transistor, and a collector of the high-frequency power transistor is a high-frequency energy output end or connected to a high-frequency energy output end; and
the high-frequency energy output end outputs high-frequency energy.

Preferably, the amplification unit includes: a power transistor and a transformer; and
a base of the power transistor is connected to the output end of the control circuit, to receive a high-frequency driving signal generated by the control circuit; and a collector of the power transistor is connected to a first end of a primary coil of the transformer, and a first end of a secondary coil of the transformer is connected to the base of the high-frequency power transistor.

Preferably, the high-frequency circuit for a low-temperature biopsy further includes: a first capacitor, a second capacitor, a first current-limiting resistor, and a second current-limiting resistor, wherein
the first capacitor and the first current-limiting resistor are sequentially connected in series between the output end of the control circuit and the base of the power transistor; and
the second capacitor and the second current-limiting resistor are sequentially connected in series between the first end of the secondary coil of the transformer and the base of the high-frequency power transistor.

Preferably, the high-frequency circuit for a low-temperature biopsy further includes: a reference voltage circuit, wherein an output end of the reference voltage circuit being connected to the base of the high-frequency power transistor.

Preferably, the reference voltage circuit includes: a reference voltage providing circuit and a reference voltage filter circuit, wherein
an output end of the reference voltage providing circuit is connected to an input end of the reference voltage filter circuit, and an output end of the reference voltage filter circuit is connected to the base of the high-frequency power transistor.

Preferably, the high-frequency circuit for a low-temperature biopsy further includes: a high-frequency transformer, wherein the high-frequency transformer is connected in series between the collector of the high-frequency power transistor and the high-frequency energy output end.

Preferably, the high-frequency circuit for a low-temperature biopsy further includes: a rectifying circuit, wherein the rectifying circuit is connected in series between a secondary coil of the high-frequency transformer and the high-frequency energy output end.

Preferably, the rectifying circuit includes: a first rectifying inductor, a second rectifying inductor, a third rectifying inductor, a fourth rectifying inductor, a fifth rectifying inductor, a sixth rectifying inductor, a first rectifying capacitor, a second rectifying capacitor, a third rectifying capacitor, and a fourth rectifying capacitor, wherein
a first end of the first rectifying inductor is connected to a first end of the secondary coil of the high-frequency transformer, and a first end of the second rectifying inductor is connected to a second end of the secondary coil of the high-frequency transformer;
a second other end of the first rectifying inductor is connected to a first end of the third rectifying inductor, and a second end of the second rectifying inductor is connected to a first end of the fourth rectifying inductor;
a second end of the third rectifying inductor is connected to a first end of the fifth rectifying inductor, and a second end of the fourth rectifying inductor is connected to a first end of the sixth rectifying inductor;
a node between the first rectifying inductor and the third rectifying inductor is connected to a first end of the first rectifying capacitor, and a node between the second rectifying inductor and the fourth rectifying inductor is connected to a second end of the first rectifying capacitor;
a node between the third rectifying inductor and the fifth rectifying inductor is connected to a first end of the second rectifying capacitor, a second end of the second rectifying capacitor is connected to a first end of the third rectifying capacitor, and a node between the fourth rectifying inductor and the sixth rectifying inductor is connected to a second end of the third rectifying capacitor;
the fourth rectifying capacitor is connected in parallel to both ends of the second rectifying capacitor in parallel; and
a second end of the fifth rectifying inductor and a second end of the sixth rectifying inductor are output ends of the rectifying circuit.

Preferably, the high-frequency circuit for a low-temperature biopsy further includes: a filter circuit, wherein the filter circuit is connected in series between the output ends of the rectifying circuit and the high-frequency energy output end.

Preferably, a frequency of the high-frequency energy is greater than or equal to 1.7 MHz.

Preferably, a peak voltage of the high-frequency energy is greater than 300 Vp.

According to a second aspect of the present invention, provided is a high-frequency system for a low-temperature biopsy, which is used for a biopsy, and includes the high-frequency circuit for a low-temperature biopsy according to any one of the above items.

According to the high-frequency circuit and system for a low-temperature biopsy provided by the present invention, output of the high-frequency energy is realized through amplification of the high-frequency power transistor, which is much higher than that of an existing conventional high-frequency electrotome, not only realizes a coagulation function during cutting, but also reduces the degree of thermal damage, thereby reducing the damage to the tissue taken out for the biopsy, and reducing or avoiding the effects on a pathological analysis result.

According to the high-frequency circuit and system for a low-temperature biopsy provided by the present invention, output of the high-frequency energy with the frequency greater than 1.7 MHz is realized, which is several times that of the conventional high-frequency electrotome on the market, further reducing the degree of thermal damage.

According to the high-frequency circuit and system for a low-temperature biopsy provided by the present invention, output of the high-frequency energy with the peak voltage greater than 300 Vp is realized, and high voltage, high power and high energy are provided.

### BRIEF DESCRIPTION OF DRAWINGS

In order to explain the technical solutions in the embodiments of the present invention or in the prior art more clearly, the drawings used in the description of the embodiments or the prior art will be briefly introduced below. Clearly, the drawings in the following description are merely some embodiments of the present invention. For those of ordinary skill in the art, other drawings can be obtained based on these drawings without creative efforts.
FIG. 1 is a schematic diagram of a high-frequency circuit for a low-temperature biopsy according to an embodiment of the present invention;
FIG. 2 is a schematic diagram of a rectifying and filter circuit of a high-frequency circuit for a low-temperature biopsy according to an embodiment of the present invention; and
FIG. 3 is a diagram of comparison between thermal damage depths of a high-frequency circuit for a low-temperature biopsy according to an embodiment of the present invention and the prior art.

### DESCRIPTION OF EMBODIMENTS

The technical solutions in embodiments of the present invention will be clearly and fully described in combination with the drawings of the embodiments of the present invention; it is clear that the described embodiments are only a part of, and not all embodiments of, present invention. Based on the embodiments of the present invention, all other embodiments obtained by those of ordinary skill in the art without creative efforts should fall within the protection scope of the present invention.

In the description of the specification of the present invention, it should be understood that the term "upper portion", "lower portion", "upper end", "lower end", "upper surface", "lower surface" or the like indicates an orientation or positional relationship based on that shown in the drawings, which is merely for ease of description and simplicity of description, and is not intended to indicate or imply that the apparatus or element referred to must have a particular orientation, be constructed and operate in a particular orientation, and therefore cannot be construed as a limitation on the present invention.

In the description of the specification of the present invention, the terms "first" and "second" are used for descriptive purposes only, and cannot be construed as indicating or implying relative importance or implicitly indicating the number of technical features indicated. Therefore, a feature defined with "first" and "second" may explicitly or implicitly include one or more of the features.

In the description of the present invention, "a plurality of means multiple, such as two, three, four or the like, unless specifically defined otherwise.

In the description of the specification of the present invention, unless otherwise expressly specified and limited, the terms "connect" and the like should be broadly understood, for example, they may be a fixed connection or a detachable connection or be integrated; or may be a mechanical connection or an electrical connection, or may communicate with each other; or may be a direct connection or an indirect connection through an intermediate medium, or may be a communication between the interior of two elements or the interaction of two elements. For those of ordinary skill in the art, the specific meanings of the above terms in the present invention can be understood according to specific situations.

The technical solutions of the present invention will be described in detail below by specific embodiments. The following several specific embodiments may be mutually combined, and same or similar concepts or processes may not be repeatedly described in some embodiments.

In an embodiment, provided is a high-frequency circuit for a low-temperature biopsy, including: a control circuit and an amplification circuit. The amplification circuit includes: an amplification unit and a high-frequency power transistor. An input end of the amplification unit is connected to an output end of the control circuit, to receive a high-frequency driving signal generated by the control circuit. An output end of the amplification unit is connected to a base of the high-frequency power transistor. A collector of the high-frequency power transistor is a high-frequency energy output end or connected to a high-frequency energy output end. The high-frequency power transistor converts a high-frequency weak electrical signal into high-frequency energy. The high-frequency energy output end outputs the high-frequency energy.

At present, two main products on the market use mechanical cutting, which have complicated structures, high machining process requirements and high manufacturing costs, and mechanical cutting will inevitably lead to bleeding, which has always been a problem in the market. A high-frequency electrotome can have a coagulation function in a cutting process, which can solve this problem in the market. However, although a traditional high-frequency electrotome has the function of electric cutting and coagulation, but a thermal damage depth is large, which may damage taken-out tissue and affect a pathological result.

According to the high-frequency circuit for a low-temperature biopsy according to the above embodiment, the coagulation function can be provided during cutting, and with the usage of the high-frequency driving signal, output of high-frequency sinusoidal alternating energy and voltage is realized through the amplification of the high-frequency power transistor. The output high-frequency energy is far greater than a frequency of a traditional conventional high-frequency electrotome. Therefore, the degree of thermal damage is far less than that of the traditional conventional high-frequency electrotome, the damage to the removed tissue is avoided, thereby avoiding the effects on the pathological analysis result.

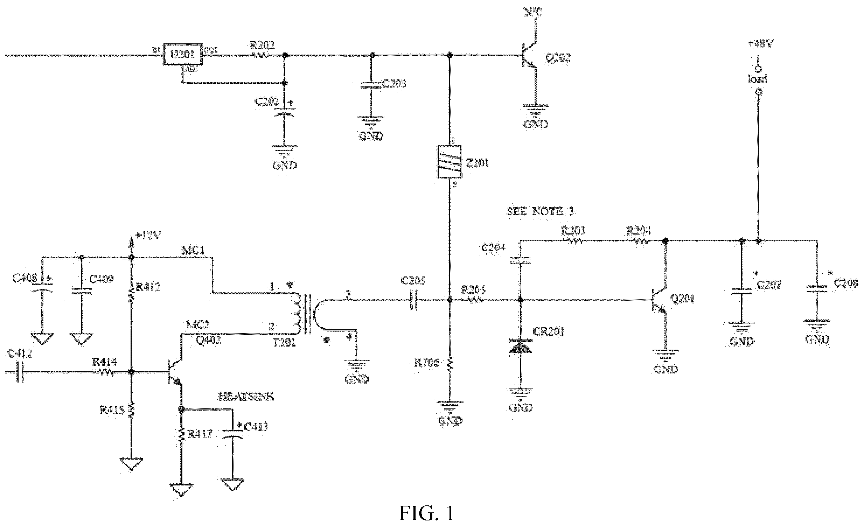

In an embodiment, the amplification unit includes: a power transistor Q402 and a transformer T201. Refer to FIG. 1. A base of the power transistor Q402 is connected to an output end of the control circuit (not shown in the figure), to receive a high-frequency driving signal generated by the control circuit. A collector of the power transistor Q402 is connected to a first end of a primary coil of the transformer T201. A first end of a secondary coil of the transformer T201 is connected to a base of a high-frequency power transistor Q201.

In an embodiment, the high-frequency circuit for a low-temperature biopsy further includes: a first capacitor C412, a second capacitor C205, a first current-limiting resistor R414 and a second current-limiting resistor R205. Refer to FIG. 1.

The first capacitor C412 and the first current-limiting resistor R414 are sequentially connected in series between the output end of control circuit and the base of power transistor Q402. The second capacitor C205 and the second current-limiting resistor R205 are sequentially connected in series between a first end of the secondary coil of the transformer T201 and the base of the high-frequency power transistor Q201.

In an embodiment, the high-frequency circuit for a low-temperature biopsy further includes: a reference voltage circuit, an output end of the reference voltage circuit being connected to the base of the high-frequency power transistor, which can ensure that the high-frequency power transistor works in an amplification area.

In an embodiment, the reference voltage circuit includes: a reference voltage providing circuit U201 and a reference voltage filter circuit Z201. Refer to FIG. 1. An output end of the reference voltage providing circuit U201 is connected to an input end of the reference voltage filter circuit Z201, and an output end of the reference voltage filter circuit Z201 is connected to the base of high-frequency power transistor Q201.

In an embodiment, the reference voltage filter circuit Z201 uses magnetic beads. In a different embodiment, the other filter element or filter circuit may also be used.

The power transistor Q402, the transformer T201, the high-frequency power transistor Q201, and the reference voltage providing circuit U201 may further include corresponding peripheral circuits. Certainly, the peripheral circuits are not necessarily the same as those in FIG. 1, and those skilled in the art can make different configurations as needed.

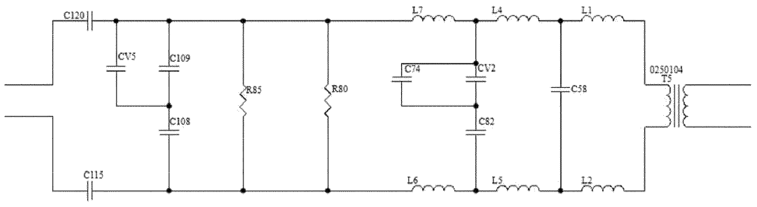

In an embodiment, the high-frequency circuit for a low-temperature biopsy further includes: a high-frequency transformer T5, the high-frequency transformer T5 being connected in series between a collector of the high-frequency power transistor Q201 and the high-frequency energy output end. Refer to FIG. 2.

In an embodiment, the high-frequency circuit for a low-temperature biopsy further includes: a rectifying circuit, the rectifying circuit being connected in series between a secondary coil of the high-frequency transformer T5 and the high-frequency energy output end.

In an embodiment, the rectifying circuit includes: a first rectifying inductor L1, a second rectifying inductor L2, a third rectifying inductor L4, a fourth rectifying inductor L5, a fifth rectifying inductor L7, a sixth rectifying inductor L6, a first rectifying capacitor C58, a second rectifying capacitor CV2, a third rectifying capacitor C82, and a fourth rectifying capacitor C74. Refer to FIG. 2.

A first end of the first rectifying inductor L1 is connected to a first end of the secondary coil of the high-frequency transformer T5, and a first end of the second rectifying inductor L2 is connected to a second end of the secondary coil of the high-frequency transformer T5. A second end of the first rectifying inductor L1 is connected to a first end of the third rectifying inductor L4, and a second end of the second rectifying inductor L2 is connected to a first end of the fourth rectifying inductor L5. A second end of the third rectifying inductor L4 is connected to a first end of the fifth rectifying inductor L7, and a second end of the fourth rectifying inductor L5 is connected to a first end of the sixth rectifying inductor L6. A node between the first rectifying inductor L1 and the third rectifying inductor L4 is connected to a first end of the first rectifying capacitor C58, and a node between the second rectifying inductor L2 and the fourth rectifying inductor L5 is connected to a second end of the first rectifying capacitor C58. A node between the third rectifying inductor L4 and the fifth rectifying inductor L7 is connected to a first end of the second rectifying capacitor CV2, a second end of the second rectifying capacitor CV2 is connected to a first end of the third rectifying capacitor C82, and a node between fourth rectifying inductor L5 and the sixth rectifying inductor L6 is connected to a second end of the third rectifying capacitor C82. The fourth rectifying capacitor C74 is connected in parallel to both ends of the second rectifying capacitor CV2. A second end of the fifth rectifying inductor L7 and a second end of the sixth rectifying inductor L6 are output ends of the rectifying circuit.

Certainly, in a different embodiment, the rectifying circuit may not be exactly the same as that in FIG. 2, and modifications can be made by those skilled in the art as needed.

In an embodiment, the high-frequency circuit for a low-temperature biopsy further includes: a filter circuit, the filter circuit being connected in series between the output ends of the rectifying circuit and the high-frequency energy output end.

In an embodiment, the filter circuit includes: a first filter resistor R80, a second filter resistor R85, a first filter capacitor C108, a second filter capacitor C109, a third filter capacitor C120, a fourth filter capacitor C115, and a fifth filter capacitor CV5. Refer to FIG. 2.

A first end of the first filter resistor R80, a first end of the second filter resistor R85, a first end of the second filter capacitor C109, and a first end of the third filter capacitor C120 are separately connected to one output end of the rectifying circuit. A second end of the first filter resistor R80, a second end of the second filter resistor R85, a first end of the first filter capacitor C108, and a first end of the fourth filter capacitor C115 are separately connected to the other output end of the rectifying circuit. A second end of the second filter capacitor C109 is connected to a second end of the first filter capacitor C108. The fifth filter capacitor CV5 is connected in parallel to both ends of the second filter capacitor C109. A second end of the third filter capacitor C120 and a second end of the fourth filter capacitor C115 are the high-frequency energy output end.

Certainly, in a different embodiment, the filter circuit may not be exactly the same as that in FIG. 2, and modifications can be made by those skilled in the art as needed.

In an embodiment, a frequency of the high-frequency energy is greater than or equal to 1.7 MHz, preferably, may be 1.7 MHz to 4 MHz. A frequency of the conventional high-frequency electrotome on the market is generally several hundred KHz, and the frequency of this embodiment is as high as 1.7MHz to 4MHz, which is several times that of the conventional high-frequency electrotome on the market. This embodiment not only has the coagulation function, but also can further reduce the damage depth, and further avoid damaging the tissue and not affecting the pathological analysis result.

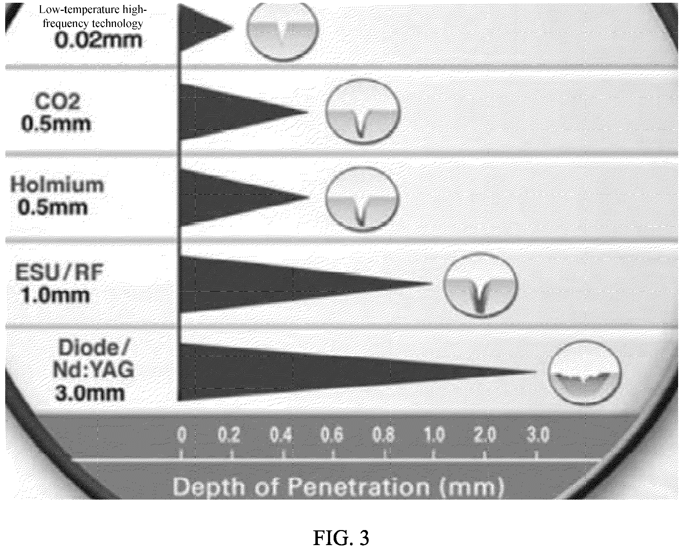

Refer to FIG. 3, which is a diagram of comparison between thermal damage depths of a high-frequency circuit for a low-temperature biopsy according to the present invention and the prior art. A high-frequency cutting technology according to the embodiment of the present invention has a thermal damage depth of 0.02 mm, a CO2 cutting technology has a thermal damage depth of 0.5 mm, a Holmlum cutting technology has a thermal damage depth of 0.5 mm, an ESU/RF cutting technology has a thermal damage depth of 1.0 mm, and a Diode/Nd:YAG cutting technology has a thermal damage depth of 3.0 mm. It can be seen from the comparison diagram that the thermal damage depth of the high-frequency cutting technology according to the embodiment of the present invention is the lowest.

In an embodiment, a peak voltage of the high-frequency energy is greater than 300 Vp, preferably, may be 300 Vp to 600 Vp, and the energy density is extremely high.

According to a second aspect of the present invention, provided is a high-frequency system for a low-temperature biopsy, which is used for a biopsy, and includes the high-frequency circuit for a low-temperature biopsy according to any one of the above items.

In the description of this specification, description of reference terms such as "an implementation", "an embodiment", "a specific implementation process", or "an example" means including specific features, structures, materials, or characteristics described in the embodiment or example in at least one embodiment or example of the present invention. In this specification, exemplary descriptions of the foregoing terms do not necessarily refer to the same embodiment or example. Furthermore, specific features, structures, materials, or characteristics described may be combined in any suitable manner in one or more embodiments or examples.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without making the essence of the corresponding technical solutions departing from the scope of the technical solutions of the embodiments of the present invention.

## Claims

1. A high-frequency circuit for a low-temperature biopsy, comprising a control circuit and an amplification circuit, wherein
the amplification circuit comprises: an amplification unit and a high-frequency power transistor;
an input end of the amplification unit is connected to an output end of the control circuit, to receive a high-frequency driving signal generated by the control circuit;
an output end of the amplification unit is connected to a base of the high-frequency power transistor, and a collector of the high-frequency power transistor is a high-frequency energy output end or connected to a high-frequency energy output end; and
the high-frequency energy output end outputs high-frequency energy.

2. The high-frequency circuit for a low-temperature biopsy according to claim 1, wherein the amplification unit comprises: a power transistor and a transformer; and
a base of the power transistor is connected to the output end of the control circuit, to receive a high-frequency driving signal generated by the control circuit; and a collector of the power transistor is connected to a first end of a primary coil of the transformer, and a first end of a secondary coil of the transformer is connected to the base of the high-frequency power transistor.

3. The high-frequency circuit for a low-temperature biopsy according to claim 2, further comprising: a first capacitor, a second capacitor, a first current-limiting resistor and a second current-limiting resistor, wherein
the first capacitor and the first current-limiting resistor are sequentially connected in series between the output end of the control circuit and the base of the power transistor; and
the second capacitor and the second current-limiting resistor are sequentially connected in series between the first end of the secondary coil of the transformer and the base of the high-frequency power transistor.

4. The high-frequency circuit for a low-temperature biopsy according to claim 1, further comprising: a reference voltage circuit, wherein an output end of the reference voltage circuit is connected to the base of the high-frequency power transistor.

5. The high-frequency circuit for a low-temperature biopsy according to claim 4, wherein the reference voltage circuit comprises: a reference voltage providing circuit and a reference voltage filter circuit, wherein
an output end of the reference voltage providing circuit is connected to an input end of the reference voltage filter circuit, and an output end of the reference voltage filter circuit is connected to the base of the high-frequency power transistor.

6. The high-frequency circuit for a low-temperature biopsy according to claim 1, further comprising: a high-frequency transformer, wherein the high-frequency transformer is connected in series between the collector of the high-frequency power transistor and the high-frequency energy output end.

7. The high-frequency circuit for a low-temperature biopsy according to any one of claims 1-6, further comprising: a rectifying circuit, wherein the rectifying circuit is connected in series between the secondary coil of the high-frequency transformer and the high-frequency energy output end.

8. The high-frequency circuit for a low-temperature biopsy according to claim 7, wherein the rectifying circuit comprises: a first rectifying inductor, a second rectifying inductor, a third rectifying inductor, a fourth rectifying inductor, a fifth rectifying inductor, a sixth rectifying inductor, a first rectifying capacitor, a second rectifying capacitor, a third rectifying capacitor, and a fourth rectifying capacitor, wherein
a first end of the first rectifying inductor is connected to a first end of the secondary coil of the high-frequency transformer, and a first end of the second rectifying inductor is connected to a second end of the secondary coil of the high-frequency transformer;
a second end of the first rectifying inductor is connected to a first end of the third rectifying inductor, and a second end of the second rectifying inductor is connected to a first end of the fourth rectifying inductor;
a second end of the third rectifying inductor is connected to a first end of the fifth rectifying inductor, and a second end of the fourth rectifying inductor is connected to a first end of the sixth rectifying inductor;
a node between the first rectifying inductor and the third rectifying inductor is connected to a first end of the first rectifying capacitor, and a node between the second rectifying inductor and the fourth rectifying inductor is connected to a second end of the first rectifying capacitor;
a node between the third rectifying inductor and the fifth rectifying inductor is connected to a first end of the second rectifying capacitor, a second end of the second rectifying capacitor is connected to a first end of the third rectifying capacitor, and a node between the fourth rectifying inductor and the sixth rectifying inductor is connected to a second end of the third rectifying capacitor;
the fourth rectifying capacitor is connected in parallel to both ends of the second rectifying capacitor; and
a second end of the fifth rectifying inductor and a second end of the sixth rectifying inductor are output ends of the rectifying circuit.

9. The high-frequency circuit for a low-temperature biopsy according to claim 7, further comprising: a filter circuit, wherein the filter circuit is connected in series between the output ends of the rectifying circuit and the high-frequency energy output end.

10. The high-frequency circuit for a low-temperature biopsy according to any one of claims 1-6, wherein a frequency of the high-frequency energy is greater than or equal to 1.7 MHz.

11. The high-frequency circuit for a low-temperature biopsy according to claim 10, wherein a peak voltage of the high-frequency energy is greater than 300 Vp.

12. A high-frequency system for a low-temperature biopsy, wherein the high-frequency system is used for a biopsy, and comprises a high-frequency circuit for the low-temperature biopsy according to any one of claims 1-11.
